Europäisches Patentamt

European Patent Office

Office européen des brevets

(11) Publication number: 0 358 248
A1

# EUROPEAN PATENT APPLICATION

(21) Application number: 89201855.7

(22) Date of filing: 13.07.89

(51) Int. Cl.5: C12Q 1/68 , C12N 15/00

(30) Priority: 15.07.88 NL 8801805

(43) Date of publication of application:
14.03.90 Bulletin 90/11

(84) Designated Contracting States:
ES GR

(71) Applicant: Rijksuniversiteit te Leiden
Stationsweg 46
NL-2312 AV Leiden(NL)

(72) Inventor: van de Klundert, Joseph Alfons
Maria
Boshuizerlaan 10
NL-2321 SG Leiden(NL)

(74) Representative: Smulders, Theodorus A.H.J.,
Ir. et al
Vereenigde Octrooibureaux Nieuwe Parklaan
107
NL-2587 BP 's-Gravenhage(NL)

(54) DNA sequencing method and primer suitable therefor.

(57) Dideoxy DNA sequencing method using the substantially random insertion of the γ-δ element of an F-plasmid into a starting plasmid containing the DNA to be sequenced. Fragments comprising a portion of the DNA to be sequenced and, adjacent thereto, either the γ end or the δ end of the γ-δ insert are isolated and subjected to dideoxy sequencing using an oligonucleotide having a length of at least 10 nucleotides, which is complementary to both 5' ends of the γ-δelement, as a primer. The above also covers suitable primer oligonucleotides.

FIG.2

EP 0 358 248 A1

## DNA sequencing method and primer suitable therefor.

This invention relates to a method of determining the nucleotide sequence of DNA by the dideoxy sequencing method.

The method most frequently used for determining the sequence of bases in DNA (sequencing) is at present the so-called M13-dideoxy method, developed by Messing (1) and Sanger (2). In this method, the DNA being investigated is cut into pieces by means of restriction enzymes, which pieces are each separately cloned in the RF form of bacteriophage M13-DNA.

This phage DNA has been rendered especially suitable for the purpose by introducing a "multiple-cloning site" (MCS), a piece of DNA containing recognition sites for various restriction enzymes (3). The phage DNA which now contains the DNA to be investigated is introduced by means of transformation into a suitable strain of Escherichia coli. In the bacterial cell, this DNA is subsequently multiplied, both in the double-stranded form (RF-DNA), and in the single-stranded form. This single-stranded form is packaged into new viral particles, which are continuously secreted by the bacterium. After the isolation of these viral particles, the DNA can be purified therefrom, whereafter it is suitable to serve as a template in the actual dideoxy sequencing method according to Sanger (2). Briefly, this method comprises making in four different reaction vessels four complementary copies of the single-stranded DNA being investigated, the reaction conditions being selected so that in each of the four vessels the newly-synthesized chain stops at one of the four nucleotides (A, G, C, or T), but at random sites in the chain. In each vessel there is thus formed a mixture of chains, from short to long, each being a true copy of the template DNA.

By separating these fragments, which have been labeled with 32P or 35S, on a high-resolution polyacrylamide gel, and after autoradiography, the sequence of bases of the DNA can simply be read.

Procedures for sequencing double-stranded DNA in which the cloning step in M13 can be omitted have also been described, for that matter (14).

In all cases, however, the reaction requires a starting fragment (primer) to which the chain to be newly synthesized is attached. By correct election of this primer, the start of the sequencing reaction can be uniquely defined.

In the M13 method described above, the primer commonly used is a synthetic oligonucleotide of 17 successive nucleotides, which is complementary to a piece of DNA located some distance ahead of the multiple-cloning site. Depending on the restriction enzymes used, the distance varies from 10 to 60 bases.

In addition to many positive properties, the above system also has some weaker aspects:

- cloning restriction fragments presupposes the presence of suitable restriction sites in the DNA being investigated, spaced apart a distance which can be spanned in one sequencing reaction (200 to 300 bases). In practice, this is an exception rather than the rule. If it occurs at all, it is often in exotic restriction enzymes. In addition to being often very expensive, these enzymes provide fragments which cannot be cloned in M13 until after many complex manipulations.
- To ensure that, during the cloning of restriction fragments, minute fragments are not overlooked, it is necessary to read beyond restriction sites.

This accordingly requires a second set of restriction fragments which overlaps the existing fragments.

- In the sequencing reaction, a piece of M13 DNA is always read first. Depending on the restriction enzyme used for the cloning, the length of this "ballast" DNA ranges from 10 to 60 nucleotides. As a consequence, the effective length read is shortened by about the same number of bases.
- It is conventional to read both strands of the DNA being investigated to minimize the risk of errors. If the fragment is not too long, the other strand can be cloned in a related M13 phage, in which the multiple cloning site is located mirror-immagewise relatively to the first pha.ge. This, in turn, requires manipulation of the DNA, renewed transformation, cultivation of phage, isolation of DNA, etc.

It is an object of the present invention to provide a method which removes these disadvantages of the known dideoxy sequencing methods.

This object is realized, in accordance with the invention, in a method of determining the nucleotide sequence of DNA by the dideoxy sequencing method, which is characterized by using as a primer an oligonucleotide of at least 10 nucleotides, which is complementary to the two 5′ ends of the γ-δ element of an F plasmid, and using as a template a restriction fragment comprising at least a portion of the DNA to be sequenced as well as one end of the γ-δ element essentially adjacent thereto, said restriction fragment being obtained by constructing a plurality of different recombinant plasmids each containing a γ-δ insert, starting from a starting plasmid containing the DNA to be sequenced, by means of transformation of bacteria containing an F plasmid, followed by conjugation with F⁻ bacteria, determining the site and orientation of the γ-δ insert by means of restriction enzyme analysis, and isolating from one or more of the recombinant plasmids a restriction fragment comprising the γ end and essentially adjacent DNA to be sequenced, and/or a restriction fragment comprising the δ end and essentially adjacent DNA to be sequenced.

More specifically, the object contemplated is realized according to this invention in a method of determining the nucleotide sequence of DNA by the M13 dideoxy sequencing method, which is characterized by using as a primer an oligonucleotide of at least 10 nucleotides, which is complementary to the two 5' ends of the γ-δ element of an F plasmid, and in that the restriction fragment inserted into M13 DNA comprises at least a portion of the DNA to be sequenced and an essentially adjacent end of the γ-δ element, which restriction fragment has been obtained by constructing a plurality of different recombinant plasmids each containing a γ-δ insert, starting from a starting plasmid containing the DNA to be sequenced, by means of transformation of bacteria containing an F plasmid, followed by conjugation with F⁻ bacteria, determining the place and orientation of the γ-δ insert by means of restriction enzyme analysis, and isolating from one or more of the recombinant plasmids a restriction fragment comprising the γ end and essentially adjacent DNA to be sequenced, and/or a restriction fragment comprising the δ end and essentially adjacent DNA to be sequenced.

The γ-δ element is a DNA fragment of 5.7 kbp forming part of an F plasmid. Such an F plasmid is a large, circular, double-stranded DNA molecule which occurs in bacteria independently of the chromosome, and can maintain itself therein independently. In addition, an F plasmid contains all information necessary to transfer itself to other (F⁻)-bacteria by means of conjugation (4).

During this conjugation, other plasmids present in the cell, which by themselves are not transferable, can be mobilized so that they, too, are transferred. Such mobilized plasmids turn out to be all enriched, after the transfer, with a fragment of DNA from the F plasmid, namely, the γ-δ element. This element behaves as a transposon: it has the property of integrating into the recipient DNA at random places, but only once per molecule.

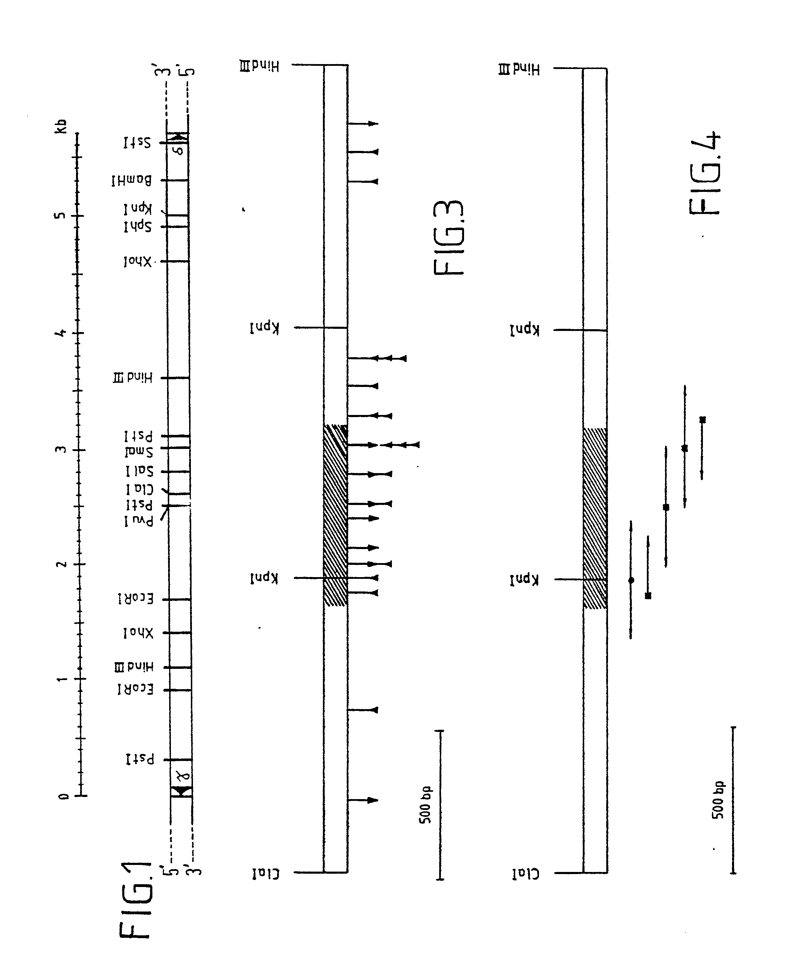

The two ends of the γ-δ element form so-called "inverted repeats" of 35 to 40 basepairs. As the name implies, the sequences of these ends are identical, but of opposite polarity (5). Adjacent to the ends of the γ-δ element, there are recognition sites or restriction enzymes, which also occur in the multiple-cloning site of M13-DNA (see Fig. 1). These properties are utilized, according to this invention in the sequencing of DNA (see Fig. 2). For this purpose, a plasmid containing the DNA to be investigated is introduced into an Escherichia coli strain containing an F plasmid. By means of conjugation, the plasmid is transferred to a different strain, whereby a large number of clones is obtained, all containing the plasmid to be investigated, each with a γ-δ insert at a different location. By isolating the plasmid DNA and cleaving with restriction enzymes, the position and orientation of the insert can be determined by means of a comparison with the restriction profiles of the starting plasmid. In the light of this information, a number of insert clones is selected which span the DNA to be sequenced. In many cases these will be clones in which the γ-δ insert is located within the area of the DNA to be sequenced. The known restriction sites in the γ-δ sequence are now used for isolating fragments, which are cloned in M13. Subsequently, the dideoxy sequencing technique is applied to these templates, using as the primer a synthetic oligonucleotide which is complementary to the two 5' ends of the γ - δ sequence. As this sequence is directly adjacent to the insertion site, the unknown DNA is directly read-in without any "ballast" DNA. If, as will mostly be the case, the insert is located within the DNA being investigated, both ends can be used for the sequencing reactions. By reason of the character of the "inverted repeats", the same oligonucleotide can be used as a primer therein.

The oligonucleotide to be used as a primer, which is also covered per se by the present invention, should have a length of at least 10, preferably at least 15 nucleotides. An oligonucleotide having the formula 5'-TTCCATTGGCCCTCAAACCC-3' has been found to be very suitable as a primer. Naturally, an oligonucleotide of less than, or more than these 20 nucleotides can be used, provided it satisfies the requirements of a minimum length of 10 nucleotides and complementarity to both 5' ends of the γ-δ element sequence. Methods of chemical synthesis of oligonucleotides are known to those skilled in the art.

A preferred embodiment of the method according to the invention is characterized by isolating from a recombinant plasmid both a restriction fragment comprising the γ end and essentially adjacent DNA to be sequenced, and a restriction fragment comprising the δ end and essentially adjacent DNA to be sequenced, and cloning one of the two restriction fragments in M13-mp18-RF DNA, and the other restriction fragment in M13-mp19-RF DNA.

Instead of the M13 mp18 and M13 mp19 vectors, other pairs of vectors with a multiple-cloning site in opposite orientation can be used. Suitable vectors are commercially available.

From practical considerations, it may be attractive to omit the cloning step in M13. The restriction fragments with either the γ end or the δ end could then be directly isolated from the gel and sequenced. At present, however, the above procedure, including cloning in M13 is preferred.

The here described use of γ-δ inserts for constructing suitable templates for sequencing DNA by the dideoxy method according to Sanger adds a new dimension to the existing technique. The dependence on suitable restriction sites in the DNA being sequenced is thus overcome. The restriction sites are, so to say, created by the insertion. By virtue of this, the construction of suitable templates is greatly simplified. In addition, the use of the ends of the inserts as a starting site for the sequencing enhances the efficiency of the method.

The utility of γ-δ inserts as starting sites for the sequencing of DNA stands or falls with the randomness with which such an insertion takes place. Results hitherto obtained indicate a large degree of randomness and hence universal applicability of the invention here described.

The invention will now be elucidated in more detail by means of a description of the accompanying drawings and an example.

Description of the drawings

Fig. 1. Physical map of the γ-δ element The triangles indicate the "inverted repeats" (γ and δ) at the two ends. The map has been composed using data of Guyer (4), Goto et al (5) and our own results.

Fig. 2. Flow sheet of the procedure followed: the base sequence of the dark portion of the recombinant plasmid has to be determined. For this purpose it is transformed (1) to a bacterium containing an F plasmid. The spontaneous γ-δ inserts (2) are isolated after conjugation (3). After plasmid isolation (4) the position of the γ-δ insert is determined by means of restriction enzymes (5). Suitable restriction fragments are purified and, whether or not cloned in M13 bacteriophage, sequenced by the didesoxy method (6).

Fig. 3. Physical map of the cloned 2.8 kb HindIII-ClaI fragment. The hatched portion is the area of interest. The triangles indicate the site and orientation of the γ-δ inserts (▲ :γ → δ; ▼ : δ→γ ).

Fig. 4. Diagrammatic representation of the sequencing strategy followed for the hatched area.

←——————•——————→ :

sequenced after the cloning of KpnI fragments in M13, without the mediation of γ-δ inserts.

←——————■——————→ :

sequenced after γ-δ insertions and cloning of suitable restriction fragments in M13.

Fig. 5. Diagrammatic representation of the cloning and sequencing procedures from one of the γ-δ inserts. The area adjacent to the δ end was sequenced, after cloning of the relevant SstI-KpnI fragment in M13 mp19; the area adjacent to the γ end after cloning of the PstI-KpnI fragment in M13 mp18. The dotted line indicates γ-δ DNA. The starting site of the sequencing reaction at the two ends is designated by ◁ p (=primer). Note the difference in scale between the various elements.

EXAMPLE

A. Introduction

An existing study is directed to a fragment of DNA of Enterobacter cloacae, which was cloned in the vector plasmid pACYC184. Transformants with the recombinant plasmid, called pJS04, turn out to have a decreased sensitivity to beta-lactam antibiotics. This insensivity is accompanied by the production of a 20 kDa protein, the information for which is located on the cloned DNA fragment (6). To determine the nature of the 20 kDa protein, it was decided to sequence the cloned gene. It turned out to be impossible, however, for the cloned fragment to be fully digested for the M13 dideoxy sequencing method with the known restriction sites.

It is here described how γ - δ insertion mutants were constructed and used for sequencing the gene.

B. Material and Methods

(i) Bacterial strains:
- Escherichia coli CE 1304: F′(lac⁺); Str$^R$, RIF$^R$
Reference: De Geus et al., 1983 (7)
- Escherichia coli W 3110: F⁻, Nal$^R$, Rif$^R$
Reference: Campbell et al., 1978 (8)
- Escherichia coli KMBL 5071: F′(lacI$^q$,Z$_{M15}$,pro); ara, Δ pro-lac, thi
Reference: Brouwer, 1988 (9)
(ii) Plasmid:
pJSO4: pACYCL184: 2.8 kb HindIII-ClaI fragment; Cam$^R$, Cpt$^R$
Reference: Stoorvogel et al., 1987 (6)
(iii) Bacteriophages:
M13mp18 and M13mp19; contain the multiple-cloning site in opposite orientation.
Reference: Yanisch-Perron et al., 1985 (3)
(iv) Transformation:
The transformation of competent cells of Escherichia coli CE1304 by the plasmid pJS04 was carried out as described in Maniatis et al. (10). Transformants were selected on "Brain-Heart Infusion" agar (Oxoid, CM 375) containing chloramphenicol (34 mg.1$^{-1}$).

(v) Conjugation

Conjugative transfer of plasmid DNA of the Escherichia coli strain CE 1304 to the strain W3110 was effected in suspension, as described in Miller (11). Exconjugants were selected on BHI-agar, containing nalidixic acid (100 mg.1$^{-1}$) and chloramphenicol (34 mg.1$^{-1}$).

(vi) DNA isolation

Plasmid DNA and M13-RF DNA was isolated by the method of Birnboim and Doly (12). Single-stranded M13 DNA was isolated from purified viral particles as described in Davis et al. (13).

(vii) Isolation and cloning of restriction fragments: Restriction enzymes were used in accordance with suppliers' specifications (Boehringer Mannheim; Gibco-BRL). Fragments were electrophoretically separated in agarose gels and purified therefrom by means of Gene-clean™, in accordance with manufacturer's specifications (BIO-101). Ligation of restriction fragments in the "multiple-cloning site" of M13-RF DNA was effected with the enzyme T4-DNA ligase in accordance with manufacturers' specifications (Boehringer, Mannheim).

(viii) Dideoxy sequencing: The dideoxy sequencing procedure was carried out in accordance with the standard instruction of Sanger (2), as described in Davis et al. (13), except for the primer used. For this purpose, an oligonucleotide (n = 20) was used, which is complementary to the two 5′ ends of the γ-δ sequence. The sequence of the oligonucleotide obtained by chemical synthesis is as follows:
5′-T-T-C-C-A-T-T-G-G-C-C-C-T-C-A-A-A-C-C-C-OH-3′

C. Results

The plasmid pJS04 to be investigated was introduced by transformation into the Escherichia coli strain CE1304, which itself contains the F plasmid. Owing to the resistance to chloramphenicol, located on pJS04, transformants could be isolated on plates containing this antibiotic. The plasmid pJS04 is, by nature, not self-transferable and also not mobilizable. If, after conjugation, it is yet found to have been transferred, this is due to the acquisition of the γ-δ element from the F plasmid.

Conjugation of the transformed strain CE 1304 with the strain W 3110, followed by selection of exconjugants on plates containing nalidixic acid (so that W 3110 is capable of growing only) and chloramphenicol (for the plasmid pJS04) did indeed provide a large number of isolates which were all found to contain the plasmid pJS04 with a γ-δ insert. To determine the location of the various inserts, plasmid DNA was isolated and analyzed by means of the restriction enzymes EcoRI, HindIII and BamHI. By comparing the restriction profiles with those of pJS04 itself, the location of the insert and its orientation could be accurately determined. As is apparent from the insert map (see Fig. 3), the inserts are scattered throughout the entire fragment. The area in which we have a special interest (hatched part of the drawing) covers about 1,000 base pairs in the centre of the cloned fragment. Through the KpnI cleavage site, a portion of the fragment is accessible for sequencing after cloning in M13-RF DNA. The major part, however, is not accessible. In this area γ-δ insert mutants were selected as indicated in Fig. 4.

To illustrate the further course, the procedure for one of the insertions will be described in detail.

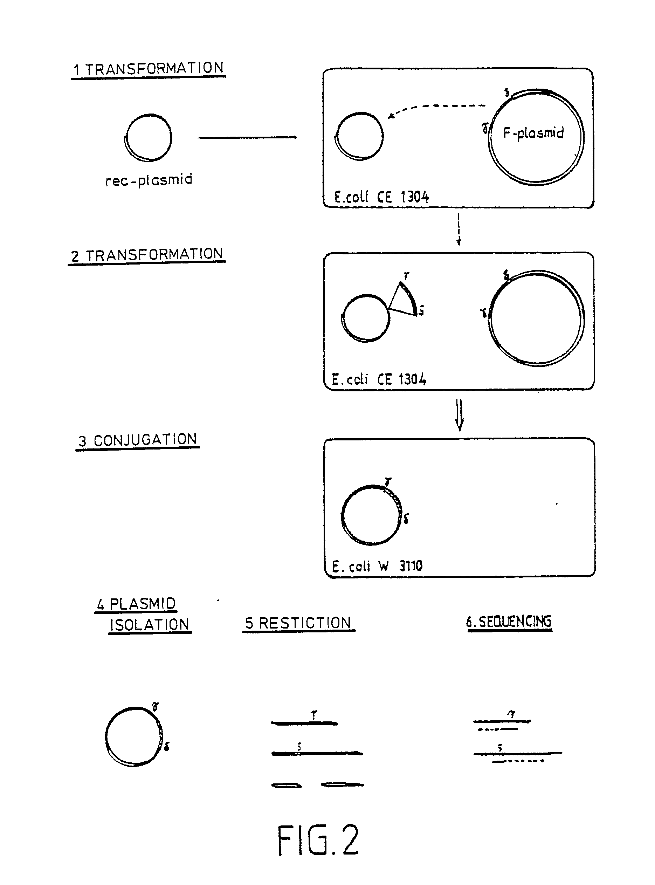

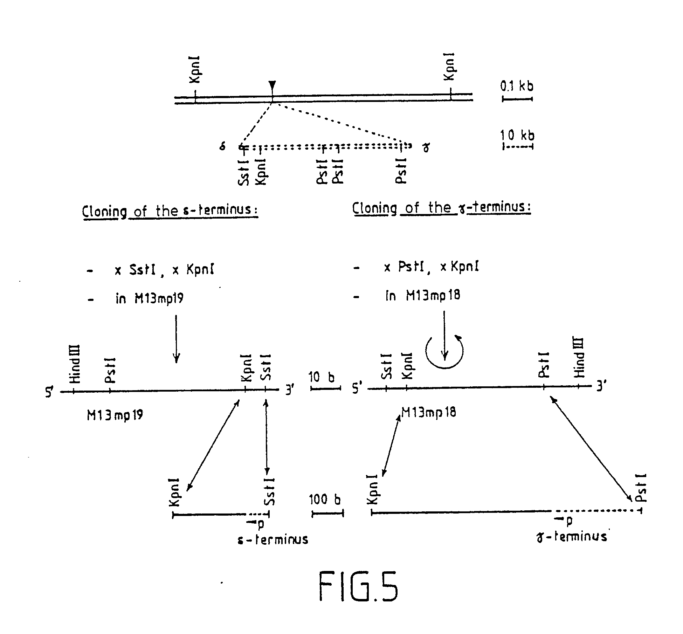

As indicated in Fig. 5, this insert is located within the unknown area, and it is desirable that both flanking pieces be sequenced. The left-hand part adjoins the δ end. Of this piece, an SstI-KpnI fragment was isolated, which was cloned in the multiple-cloning site of M13-mp19-RF DNA. The right-hand area adjoins the γ end and was cloned as a PstI-KpnI fragment in M13-mp18-RF DNA. The two re-combined RF DNAs were transferred by transformation to Escherichia coli KMBL 5071, a strain which is specially suitable for propagating the M13 bacteriophages mp18 and mp19 and derivatives thereof. After purification of the phage particles, single-stranded DNA was isolated from these, which was sequenced by the dideoxy method, using the 20n oligonucleotide, mentioned before, as a primer. In this way, the sequence of bases in the unknown DNA could be clarified from both the γ end and the δ end.

REFERENCES

1. Messing, J.; Meth.Enzymol.101, 20, 1983.
2. Sanger, F., Nicklen, S. & Coulsen, A.R.; Proc.Natl.Acad.Sci. USA, 74, 5463, 1977.
3. Yanisch-Perron, C., Vieira, J. & Messing, J.; Gene 33, 103, 1985.
4. Guyer, M.S.; J. Mol. Biol. 126, 347, 1978.
5. Goto, N. Mochizuki, A., Inagaki, Y., Horiuchi, S., Tanaka, T. & Nakaya, R.; J.Bacteriol. 169, 4388, 1987.
6. Stoorvogel, J., van Bussel, M.J.A.W.M. & Van de Klundert, J.A.M.; FEMS Microbiol. Lett. 48, 277, 1987.
7. De Geus, P., Van Die, I., Bergmans, H., Tommassen, J., De Haas, G.,; Mol.Gen.Genet. 190, 150, 1983.
8. Campbell, J., Richardson, C.C. & Studier, F.W.; Proc.Natl.Acad.Sci. USA, 75, 2276, 1978.

9. Brouwer, J.; Thesis Leiden, 1988.

10. Maniatis, T., Fritsch, E.F. & Sambrook, J.: Molecular cloning, a laboratory manual. Cold Spring Harbor Laboratory, Cold Spring Harbor, N.Y., 1982.

11. Miller, J.H.: Experiments in molecular genetics. Cold Spring Harbor Laboratory, Cold Spring Harbor, N.Y., 1972.

12. Birnboim, H.C. & Doly, J.; Nucleic Acids Res. 7, 1513, 1979.

13. Davis, L.G., Dibner, M.D., De Battey, J.F.: Basic methods in molecular biology. Elsevier, New York, Amsterdam, London, 1986.

14. Smith, A.J.H.; Nucleic Acids Res. 6, 831, 1979.

**Claims**

1. A method of determining the nucleotide sequence of DNA by the dideoxy sequencing method, characterized by using as a primer an oligonucleotide of at least 10 nucleotides, which is complementary to the two 5′ ends of the $\gamma$-$\delta$ element of an F plasmid, and using as a template a restriction fragment comprising at least a portion of the DNA to be sequenced as well as one end of the $\gamma$-$\delta$ element essentially adjacent thereto, said restriction fragment being obtained by constructing a plurality of different recombinant plasmids each containing a $\gamma$-$\delta$ insert, starting from a starting plasmid containing the DNA to be sequenced, by means of transformation of bacteria containing an F plasmid, followed by conjugation with F⁻ bacteria, determining the site and orientation of the $\gamma$-$\delta$ insert by means of restriction enzyme analysis, and isolating from one or more of the recombinant plasmids a restriction fragment comprising the $\gamma$ end and essentially adjacent DNA to be sequenced, and/or a restriction fragment comprising the $\delta$ end and essentially adjacent DNA to be sequenced.

2. A method of determining the nucleotide sequence of DNA by the M13 dideoxy sequencing method, characterized by using as a primer an oligonucleotide of at least 10 nucleotides, which is complementary to the two 5′ ends of the $\gamma$-$\delta$ element of an F plasmid, and in that the restriction fragment inserted into M13 DNA comprises at least a portion of the DNA to be sequenced and an essentially adjacent end of the $\gamma$-$\delta$ element, which restriction fragment has been obtained by constructing a plurality of different recombinant plasmids each containing a $\gamma$-$\delta$ insert, starting from a starting plasmid containing the DNA to be sequenced, by means of transformation of bacteria containing an F plasmid, followed by conjugation with F⁻ bacteria, determining the place and orientation of the $\gamma$-$\delta$ insert by means of restriction enzyme analysis, and isolating from one or more of the recombinant plasmids a restriction fragment comprising the $\gamma$ end and essentially adjacent DNA to be sequenced, and/or a restriction fragment comprising the $\delta$ end and essentially adjacent DNA to be sequenced.

3. A method as claimed in claim 2, characterized by isolating from a recombinant plasmid both a restriction fragment comprising the $\gamma$ end and essentially adjacent DNA to be sequenced, and a restriction fragment comprising the $\delta$ end and essentially adjacent DNA to be sequenced, and cloning one of the two restriction fragments in M13-mp18-RF DNA, and the other restriction fragment in M13-mp19-RF DNA.

4. A method as claimed in any of claims 1-3, characterized by using as the primer an oligonucleotide complementary to the two 5′ ends of the $\gamma$-$\delta$ element of an F plasmid and having a length of at least 15 nucleotides.

5. A method as claimed in any of claims 1-4, characterized by using as the primer an oligonucleotide having the formula 5′-TTCCATTGGCCCTCAAACCC-3′.

6. An oligonucleotide of at least 10 nucleotides, which is complementary to the two 5′ ends of the $\gamma$-$\delta$ element of an F plasmid.

7. An oligonucleotide of at least 15 nucleotides, which is complementary to the two 5′ ends of the $\gamma$-$\delta$ element of an F plasmid.

8. An oligonucleotide having the formula 5′-TTCCATTGGCCCTCAAACCC-3′.

FIG.1

FIG.3

FIG.4

EP 0 358 248 A1

FIG.2

FIG.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 209 204 (STICHTING KATHOLIEKE UNIVERSITEIT NIJMEGEN) <br> * Page 2, line 5 - page 10, line 14; page 23, line 8 - page 24, line 9 * <br> --- | 1,2,4,6 ,7 | C 12 Q 1/68 <br> C 12 N 15/00 |
| X | METHODS IN ENZYMOLOGY, vol. 152, 1987, GUIDE TO MOLECULAR CLONING TECHNIQUES, pages 538-556, Academic Press, Inc., US; W.M. BARNES: "Sequencing DNA with dideoxyribonucleotides as chain terminators: hints and strategies for big projects" <br> * Page 553, line 14 - page 554, line 26 * <br> --- | 1,2,4,6 ,7 | |
| X,P | GENE, vol. 71, no. 1, 1988, pages 193-199, Elsevier Science Publishers B.V., Amsterdam, NL; A. HIRAIWA et al.: " Locus-specific vector/primer systems for rapid coning of allelic variants" <br> * Whole article * <br> --- | 3 | |
| X | GENE, vol. 15, no. 2-3, 1981, pages 257-271, Elsvier/North-Holland Biomedical Press, Amsterdam, NL; T. MUROTSU et al.: "Nine unique repeating sequences in a region essential for replication and incompatibility of the mini-F plasmid" <br> * Whole article * <br> ---        -/- | 4-8 | TECHNICAL FIELDS SEARCHED (Int. Cl.5) <br><br> C 12 Q <br> C 12 N |

The present search report has been drawn up for all claims

| Place of search | Date of completion of the search | Examiner |
|---|---|---|
| THE HAGUE | 07-11-1989 | VAN BOHEMEN C.G. |

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | DNA, vol. 3, no. 4, 1984, pages 339-343, Mary Ann Liebert, Inc., New York, US; R. SANCHEZ-PESCADOR et al.: "Laboratory Methods; Use of unpurified synthetic deoxynucleotide primers for rapid dideoxynucleotide chain temination sequencing" * Whole article * | 1-8 | |
| Y | JOURNAL OF MOLECULAR BIOLOGY, vol. 107, no. 3, 1976, pages 207-222, Academic Press, London, GB; R.C. DEONIER et al.: "The sequence organization of the integrated F-plasmid in two Hfr strains of Escherichia coli" * Whole article * | 1-8 | |
| Y | ANALYTICAL BIOCHEMISTRY, vol. 143, 1984, pages 298-303, Academic Press, Inc., US; R.A. McGRAW: "Dideoxy DNA sequencing with end-labeled oligonucleotide primers" * Whole article * | 1-4,6,7 | |
| Y | MGG-MOLECULAR AND GENERAL GENETICS, vol. 188, no. 3, 1982, pages 513-518, Springer-Verlag, US; R. THOMPSON et al.: "Promotor mapping and DNA sequencing of the F-plasmid transfer genes traM and traJ" * Whole article * | 1-4,6,7 | TECHNICAL FIELDS SEARCHED (Int. Cl.5) |

The present search report has been drawn up for all claims

| Place of search | Date of completion of the search | Examiner |
|---|---|---|
| THE HAGUE | 07-11-1989 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)